# EUROPEAN PATENT SPECIFICATION

(11) **EP 3 893 890 B1**
(45) Date of publication and mention of the grant of the patent: **10.12.2025**
(21) Application number: 21733035.6
(22) Date of filing: 19.02.2021
(51) Int. Cl.: A61K 31/7032, A61P 11/00, A61P 31/12, A61K 45/06

(54) **GINSENOSIDE M1 FOR USE IN THE TREATMENT OF DAMAGES IN ORGANS OR TISSUES IN A SUBJECT CAUSED BY INFECTION WITH CORONAVIRUS**
GINSENOSID M1 ZUR VERWENDUNG BEI DER BEHANDLUNG VON SCHÄDEN IN ORGANEN ODER GEWEBEN IN EINEM SUBJEKT, DAS DURCH INFEKTION MIT CORONAVIRUS VERURSACHT WIRD
GINSÉNOSIDE M1 POUR UTILISATION DANS LE TRAITEMENT DE LÉSIONS D'ORGANES OU DE TISSUS CHEZ UN SUJET PROVOQUÉES PAR UNE INFECTION PAR UN CORONAVIRUS

(30) Priority: 20.02.2020 US 202062978995 P
(43) Date of publication of application: 20.10.2021
(73) Proprietor: Lee, Sheau-Long, Taoyuan City 32056 (TW)
(72) Inventor: Lee, Sheau-Long, Taoyuan City 32056 (TW)
(74) Representative: karo IP
(86) International application number: PCT/CN2021/076808
(87) International publication number: WO 2021/164723

(56) References cited:
- EP-A1- 1 723 962
- EP-A1- 2 077 119
- WO-A1-2015/165422
- WO-A1-2015/172746
- WO-A1-2016/146079
- WO-A1-2016/146079
- CN-A- 1 679 600
- US-A1- 2016 022 751
- LIU HUI ET AL: "Ginsenoside Rg3 Attenuates Angiotensin II-Mediated Renal Injury in Rats and Mice by Upregulating Angiotensin-Converting Enzyme 2 in the Renal Tissue", vol. 2019, 29 November 2019 (2019-11-29), US, pages 1 - 11, XP055878084, ISSN: 1741-427X, Retrieved from the Internet <URL:https://www.ncbi.nlm.nih.gov/pmc/articles/PMC6915024/pdf/ECAM2019-6741057.pdf> DOI: 10.1155/2019/6741057
- CHENG FANG ET AL: "Total saponins of panax ginseng inhibiting human endothelium cells damages induced by angiotensin II via AT1 receptor", JOURNAL OF ETHNOPHARMACOLOGY, ELSEVIER IRELAND LTD, IE, vol. 138, no. 2, 18 September 2011 (2011-09-18), pages 439 - 444, XP028112733, ISSN: 0378-8741, [retrieved on 20110922], DOI: 10.1016/J.JEP.2011.09.036
- LEE CHANG-SEOP ET AL: "Preventive Effect of Korean Red Ginseng for Acute Respiratory Illness: A Randomized and Double-Blind Clinical Trial", vol. 27, no. 12, 1 January 2012 (2012-01-01), SEOUL, KR, pages 1472 - 1478, XP055879023, ISSN: 1011-8934, Retrieved from the Internet <URL:https://jkms.org/pdf/10.3346/jkms.2012.27.12.1472> DOI: 10.3346/jkms.2012.27.12.1472
- YU SHAOPENG ET AL: "Glycyrrhizic acid exerts inhibitory activity against the spike protein of SARS-CoV-2", PHYTOMEDICINE, ELSEVIER, AMSTERDAM, NL, vol. 85, 2 October 2020 (2020-10-02), XP086537982, ISSN: 0944-7113, [retrieved on 20201002], DOI: 10.1016/J.PHYMED.2020.153364
- WU C-Y ET AL: "Small molecule targeting severe acute respiratory syndrome human coronavirus", PROCEEDINGS OF THE NATIONAL ACADEMY OF SCIENCES, NATIONAL ACADEMY OF SCIENCES, vol. 101, no. 27, 6 July 2004 (2004-07-06), pages 10012 - 10017, XP002993122, ISSN: 0027-8424, DOI: 10.1073/PNAS.0403596101
- CAO, MAN ET AL.: "Advances in the study of derivatization of ginsenosides andtheir anti-tumor structure-activity relationship", ACTA PHARMACEUTICA SINICA, vol. 47, no. 7, 2012, pages 836 - 843
- PARK SEONG-EUN ET AL: "Biotransformation of major ginsenosides in ginsenoside model culture by lactic acid bacteria", JOURNAL OF GINSENG RESEARCH, vol. 41, no. 1, 1 January 2017 (2017-01-01), KR, pages 36 - 42, XP093212807, ISSN: 1226-8453, Retrieved from the Internet <URL:https://www.ncbi.nlm.nih.gov/pmc/articles/PMC5223066/pdf/main.pdf> DOI: 10.1016/j.jgr.2015.12.008
- "Molecular Biology of the SARS-Coronavirus", 1 January 2010, SPRINGER BERLIN HEIDELBERG, Berlin, Heidelberg, ISBN: 978-3-642-03683-5, article ZUO WEI, ZHAO XINGANG, CHEN YE-GUANG: "SARS Coronavirus and Lung Fibrosis", pages: 247 - 258, XP055838831, DOI: 10.1007/978-3-642-03683-5_15
- PAZYAR, N.;OMIDIAN, M.;JAMSHYDIAN, N.;: "Ginseng as a potential novel addition to the antikeloid weaponry", PHYTOTHERAPY RESEARCH, WILEY, UK, 1 March 2013 (2013-03-01), UK, pages 1579 - 1580, XP018506720, ISSN: 0951-418x, DOI: 10.1002/ptr.4598

## Description

### RELATED APPLICATIONS

This application claims the benefit of U.S. provisional application number 62/978,995, filed February 20, 2020.

### FIELD OF THE INVENTION

The present invention relates to a new use of ginsenoside M1 as a modulator of angiotensin regulating enzymes and relevant treatment methods using the same. In particular, the present invention provides a method for treating a disease or conditions associated with imbalance in angiotensin regulating enzymes, including those caused by viral infection and resulting damages in organs or tissues, using ginsenoside M1. Any references in the description to methods of treatment refer to the compounds, pharmaceutical compositions and medicaments of the present invention for use in a method for treatment of the human (or animal) body by therapy (or for diagnosis).

### BACKGROUND OF THE INVENTION

Renin-Angiotensin System (RAS) or Renin-Angiotensin-Aldosterone System (RAAS) is a hormonal system. When a large amount of blood loss or blood pressure drops, this system will be activated, which is a key hormone can regulates blood pressure and body fluid constant in the body. Both angiotensin-converting enzyme (ACE) and angiotensin-converting enzyme 2 (ACE2) play important roles in RAS. ACE2 will generate angiotensin I (Ang I ) to angiotensin (Ang)-(1-9) or Ang II to Ang-(1-7) in order to achieve fluid balance. RAS plays an important role in many cardiovascular diseases. The present studies shown that ACE2 deficiency can indues acute or chronic lung injury or even pulmonary fibrosis. On the other hand, excessive activation of Ang II can lead to blood vessels and kidney damage, and progression to necrosis and fibrosis. Therefore, the lack of ACE2 can lead to excessive Ang II accumulation can cause to the lung and kidney damage. However, the latest research shows that the S protein of the new coronavirus will bind to ACE2 and invade the lung. Excessive accumulation of Ang II in the body, resulting in pulmonary fibrosis and kidney damage phenomenon. [1]

LIU HUI ET AL reported that angiotensin II (Ang II) mediated renal injury represents a major pathogenetic mechanism in most chronic kidney diseases. The previous research demonstrated that ginsenoside Rg3 attenuates Ang II elevation in the myocardium in spontaneously hypertensive rats (SHR). It is possible that Rg3 has similar effects in the renal tissue. "Ginsenoside Rg3 Attenuates Angiotensin Il-Mediated Renal Injury in Rats and Mice by Upregulating Angiotensin-Converting Enzyme 2 in the Renal Tissue"; EVIDENCE-BASED COMPLEMENTARY AND ALTERNATIVE MEDICINE, vol. 2019, 29 November 2019 (2019-11-29), pages 1-11.

CHENG FANG ET AL made a study to investigate the effect and mechanism of total saponins of panax ginseng (TSPG) on the damages of endothelium cells induced by Angiotensin II (AngII), and showed that plasma TNF-α increased significantly in AngII group when compared with the same group, and decreased significantly in AngII + TSPC group. "Total saponins ofpanax ginseng inhibiting human endothelium cells damages induced by angiotensin II via AT1 receptor", JOURNAL OF ETHNOPHARMACOLOGY, ELSEVIER IRELAND LTD, IE, vol. 138, no. 2, 18 September 2011 (2011-09-18),pages 439-444.

WU C-Y ET AL identifies small molecules that target SARS-CoV, inhibiting viral replication by disrupting key enzymatic functions. The findings provide a foundation for antiviral drug development against coronaviruses. "Small molecule targeting severe acute respiratory syndrome human coronavirus" , PROCEEDINGS OF THE NATIONAL ACADEMY OF SCIENCES, NATIONAL ACADEMY OF SCIENCES, vol. 101, no. 27, 6 July 2004 (2004-07-06), pages 10012-10017.

Coronaviruses pose serious health threats to humans and other animals. From 2002 to 2003, severe acute respiratory syndrome coronavirus (SARS-CoV) infected 8,000 people, with a fatality rate of ~10%. Since 2012, Middle East respiratory syndrome coronavirus (MERS-CoV) has infected more than 1,700 people, with a fatality rate of ~36%. Since 2013, porcine epidemic diarrhea coronavirus (PEDV) has swept throughout the United States, causing an almost 100% fatality rate in piglets and wiping out more than 10% of America's pig population in less than a year. Further, novel coronavirus (also called SARS-CoV-2) causes the COVID-19 pandemic. As of February 6, 2021, more than 105 million cases have been confirmed with more than 2.29 million deaths attributed to COVID-19 according to the Wikipedia website.

In general, coronaviruses cause widespread respiratory, gastrointestinal, and central nervous system diseases in humans and other animals, threatening human health and causing economic loss. Coronaviruses are capable of adapting to new environments through mutation and recombination with relative ease and hence are programmed to alter host range and tissue tropism efficiently. Therefore, health threats from coronaviruses are constant and long-term. Understanding the virology of coronaviruses and controlling their spread have important implications for global health and economic stability.

Coronaviruses belong to the family Coronaviridae in the order Nidovirales. They can be classified into four genera: Alphacoronavirus, Betacoronavirus, Gammacoronavirus, and Deltacoronavirus. Among of them, alpha-and betacoronaviruses infect mammals, gammacoronaviruses infect avian species, and deltacoronaviruses infect both mammalian and avian species. Representative alphacoronaviruses include human coronavirus NL63 (HCoV-NL63), porcine transmissible gastroenteritis coronavirus (TGEV), PEDV, and porcine respiratory coronavirus (PRCV). Representative betacoronaviruses include SARS-CoV, MERS-CoV, bat coronavirus HKU4, mouse hepatitis coronavirus (MHV), bovine coronavirus (BCoV), and human coronavirus OC43. Representative gamma-and deltacoronaviruses include avian infectious bronchitis coronavirus (IBV) and porcine deltacoronavirus (PdCV), respectively. Coronaviruses are large, enveloped, positive-stranded RNA viruses. They have the largest genome among all RNA viruses, typically ranging from 27 to 32 kb. The genome is packed inside a helical capsid formed by the nucleocapsid protein (N protein) and further surrounded by an envelope. Associated with the viral envelope are at least three structural proteins: The membrane protein (M protein) and the envelope protein (E protein) are involved in virus assembly, whereas the S protein mediates virus entry into host cells. Some coronaviruses also encode an envelope-associated hemagglutinin-esterase protein (HE protein). Among these structural proteins, the spike forms large protrusions from the virus surface, giving coronaviruses the appearance of having crowns (hence their name; corona in Latin means crown). In addition to mediating virus entry, the spike is a critical determinant of viral host range and tissue tropism and a major inducer of host immune responses. [2]

Ginsenosides, the main active ingredients of ginseng, are known to have a variety of pharmacological activities, e.g. antitumor, antifatique, antiallergic and antioxidant activities. Ginsenosides share a basic structure, composed of gonane steroid nucleus having 17 carbon atoms arranged in four rings. Ginsenosides are metalized in the body, and a number of recent studies suggest that ginsenoside metabolites, rather than naturally occurring ginsenosides, are readily absorbed in the body and act as the active components. Among them, ginsenoside M1, also named Compound K (CK), is known as one metabolite of protopanaxadiol-type ginsenosides via the gypenoside pathway by human gut bacteria. Until now, no prior art references report the effect of ginsenoside M1 in regulating angiotensin regulating enzymes and its use for treatment for relevant diseases or conditions.

### BRIEF SUMMARY OF THE INVENTION

The invention is set out in the appended claims.

In the present invention, it has been unexpectedly found that ginsenoside M1 is effective in regulating angiotensin regulating enzymes, including upregulating ACE2 and downregulating ACE, promoting degrading angiotensin II and preventing accumulation of angiotensin II, and thus useful for treatment of a disease or condition associated with abnormal balance of ACE and ACE2 or accumulation of angiotensin II, particularly damages in organs or tissues caused by infection of virus e.g. coronavirus.

Therefore, the present invention provides ginsenoside M1 for use in the treatment of damages in organs or tissues in a subject matter caused by infection with coronavirus, wherein the ginsenoside M1 is used as a modulator of an angiotensin regulating enzyme in a subject in need thereof.

In some embodiments of the invention, the angiotensin regulating enzyme is selected from the group consisting of angiotensin-converting enzyme (ACE) and angiotensin-converting enzyme 2 (ACE2).

In some embodiments, the modulator is an ACE inhibitor or an ACE2 activator or both.

In some embodiments, the modulator is effective in degrading angiotensin II and preventing accumulation of angiotensin II in the subject.

In some embodiments, ginsenoside M1 as a medicament is effective in treating a disease or condition associated with imbalance in ACE and ACE2 or accumulation of angiotensin II in the subject.

In some embodiments, ginsenoside M1 is administered in an amount effective in inhibiting ACE, or activating ACE2 or both in the subject.

In some embodiments, ginsenoside M1 is administered in an amount effective in degrading angiotensin II and preventing accumulation of angiotensin II in the subject.

In some reference embodiments, the disease or condition to be treated are associated with abnormal balance of ACE and ACE2 or accumulation of angiotensin II, including damages in organs or tissues in the subject.

In some reference embodiments, the damages includes damages in lung, gastrointestinal tract, spleen, lymph nodes, heart, kidney, bladder, liver, gallbladder, adrenal glands and/or testis.

In some embodiments, the damages are caused by infection of coronavirus.

In some embodiments, the coronavirus is selected from severe acute respiratory syndrome coronavirus (SARS-CoV), middle east respiratory syndrome coronavirus (MERS-CoV), and novel coronavirus (2019-nCoV).

In some embodiments, ginsenoside M1 is administered in combination with one or more additional therapeutic methods or agents useful for degrading angiotensin II and preventing accumulation of angiotensin II. In one example, such one or more additional therapeutic agents include ACE2.

In some embodiments, ginsenoside M1 is administered in combination with one or more additional therapeutic agents selected from the group consisting of corticosteroids, non-steriodal anti-inflammatory drugs (NSAIDs), cytotoxic drugs, immunosuppressants, and vasodilators.

In some embodiments, ginsenoside M1 is administered in combination with one or more additional therapeutic agents selected from the group consisting of an antibiotic, interferon and an anti-viral agent.

The details of one or more embodiments of the invention are set forth in the description below. Other features or advantages of the present invention will be apparent from the following detailed description of several embodiments, and also from the appending claims.

### BRIEF DESCRIPTION OF THE DRAWINGS

For the purpose of illustrating the invention, there are shown in the drawings embodiments. It should be understood, however, that the invention is not limited to the preferred embodiments shown. In the drawings:

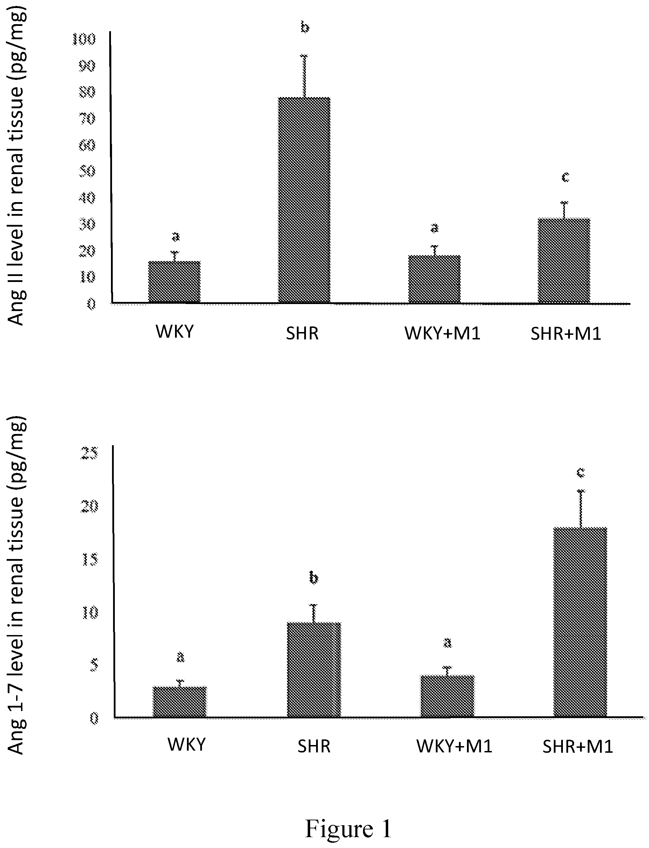

Figure 1: Angiotensin levels in the renal tissue in rats. Ang II levels in renal tissue (upper panel). Ang-(1-7) levels in renal tissue (lower panel). The same superscript letters indicate no significant difference between groups (P > 0.05); significant difference existed between groups that do not have the same superscript letter (P < 0.05).

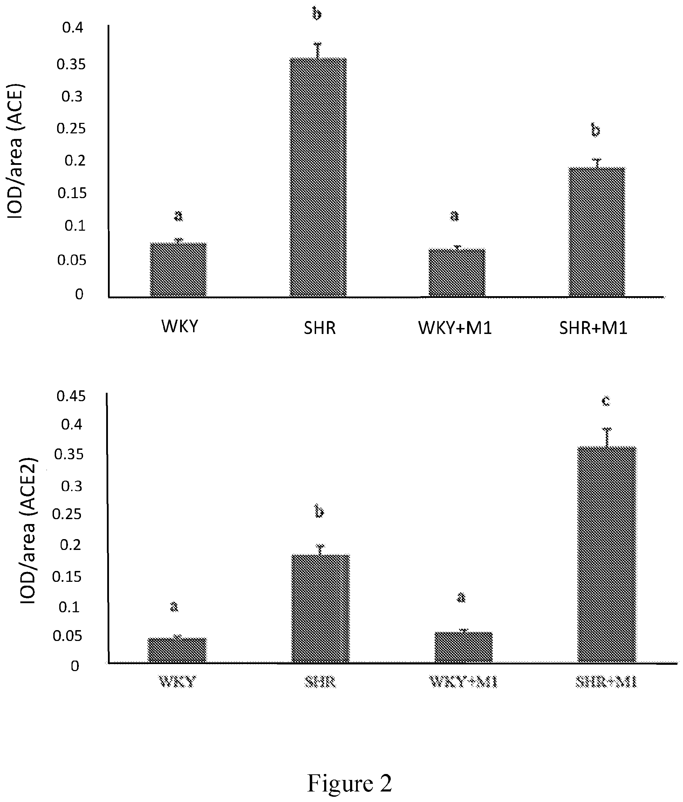

Figure 2: Levels of the angiotensin-converting enzymes in the renal tissue in rats. Quantitative results of IHC staining were presented as IOD/area and were proportional to the levels of ACE (upper panel) and ACE2 (lower panel). The same superscript letters indicate no significant difference between groups (P > 0.05); significant difference existed between groups that do not have the same superscript letter (P < 0.05).

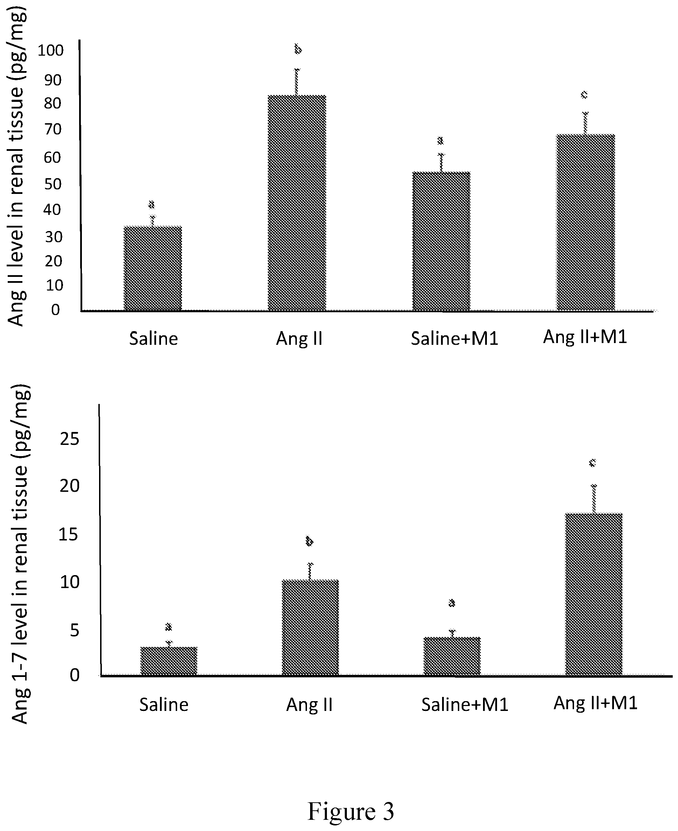

Figure 3: Angiotensins levels in the renal tissue in mice (infusion model). Ang II (upper panel) and Ang-(1-7) (lower panel) levels in the renal tissue. The same superscript letters indicate no significant difference between groups (P > 0.05); significant difference existed between groups that do not have the same superscript letter (P < 0.05).

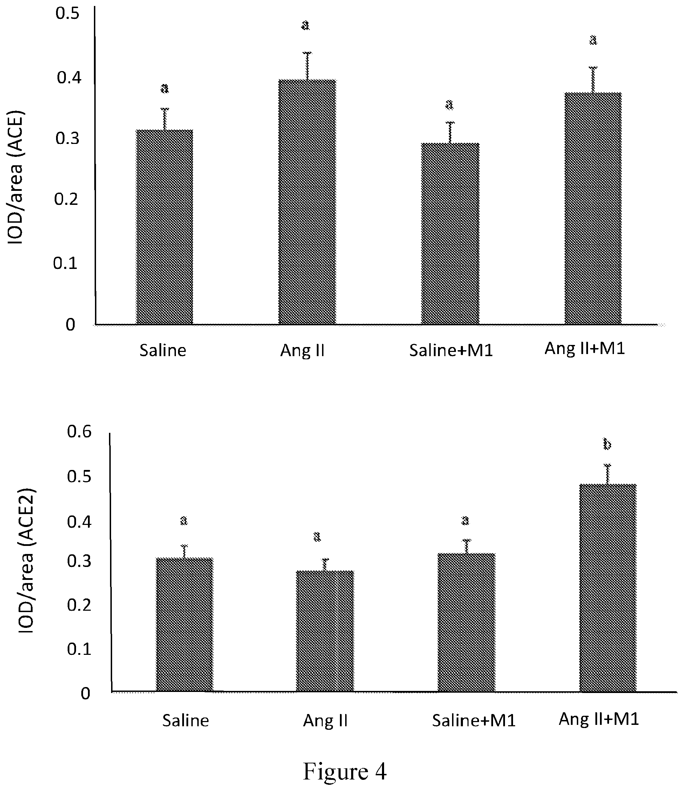

Figure 4: Levels of the ACE in the renal tissue in mice. Quantitative results of IHC staining were presented as IOD/area and were proportional to the levels of ACE (upper panel) and ACE2 (lower panel). The same superscript letters indicate no significant difference between groups (P > 0.05); significant difference existed between groups that do not have the same superscript letter (P < 0.05).

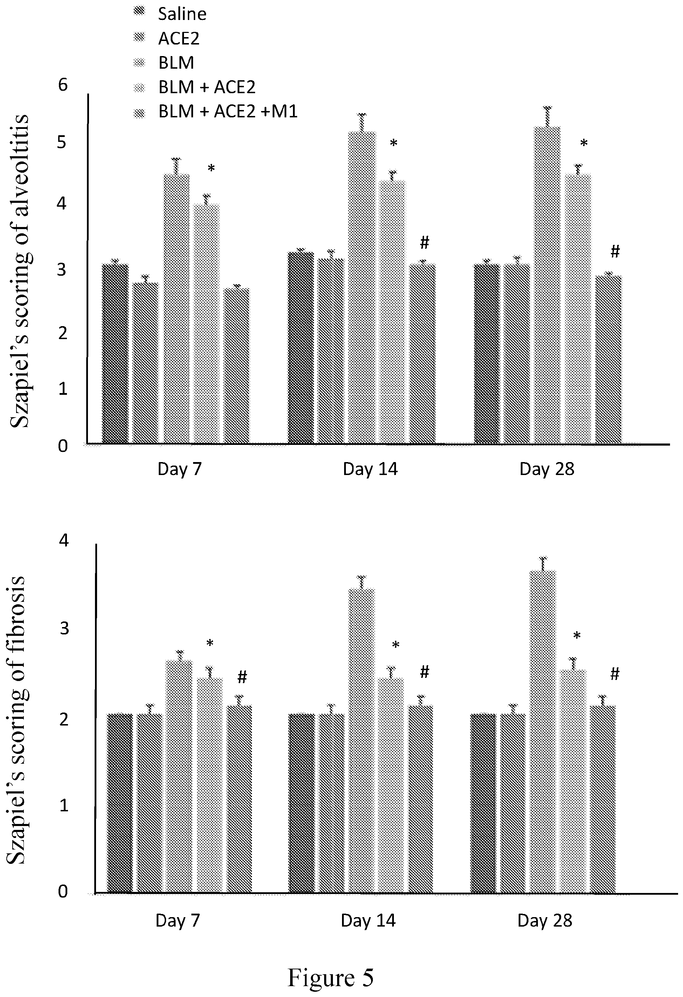

Figure 5: The local lung renin-angiotensin system. ACE2 and M1 improved histological changes in bleomycin-induced lung fibrosis. In different groups, recombinant mouse ACE2 or ACE2 together with M1 and bleomycin were administered on day 0. Szapiel's scoring was obtained to quantify the degree of alveolitis (upper panel) and fibrosis (lower panel) and inhibitory potency of ACE2 and ACE2 together with M1. Data represent as means ± SEM. *p<0.05 when compared with the Saline group, #p<0.05 when compared with BLM.

### DETAILED DESCRIPTION OF THE INVENTION

Unless defined otherwise, all technical and scientific terms used herein have the meaning commonly understood by a person skilled in the art to which this invention belongs. As used herein, the following terms have the meanings ascribed to them unless specified otherwise.

The articles "a" and "an" are used herein to refer to one or more than one (i.e., at least one) of the grammatical object of the article. By way of example, "an element" means one element or more than one element.

The term "comprise" or "comprising" is generally used in the sense of include/including which means permitting the presence of one or more features, ingredients or components. The term "comprise" or "comprising" encompasses the term "consists" or "consisting of."

Ginsenoside M1, also named Compound K (CK), 20-O-β-D-glucopyranosyl-20(S)-protopanaxadiol, is one of saponin metabolites known in the art. The chemical structure of ginsenoside M1 is as follows:

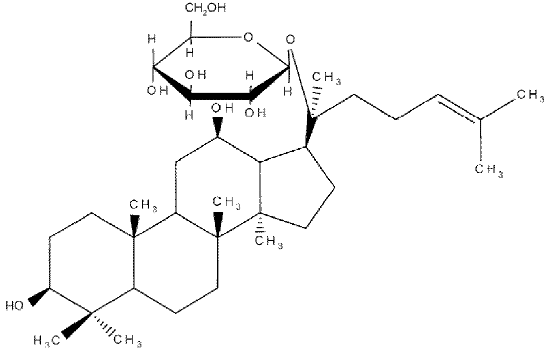

Ginsenoside M1 is known as one metabolite of protopanaxadiol-type ginsenosides via the gypenoside pathway by human gut bacteria. Ginsenoside M1 can be found in blood or urine after intake. Ginsenoside M1 may be prepared from ginseng plants through fungi fermentation by methods known in the art, such as Taiwan Patent Application No. 094116005 (I280982) and U.S. Patent No. 7,932,057. In certain embodiments, the ginseng plants for preparing the ginsenoside M1 include *Araliaceae* family, *Panax* genus, e.g. *P. ginseng* and *P. pseudo-ginseng* (also named Sanqi). In general, the method of preparation of ginsenoside M1 includes the steps of (a) providing powder of ginseng plant materials (e.g. leaves or stems); (b) providing a fungus for fermenting the ginseng plant materials, wherein the fermentation temperature is ranged from 20-50° C., the fermentation humidity is ranged from 70-100%, the pH value is ranged from 4.0-6.0, and the fermentation period is ranged from 5-15 days; (c) extracting and collecting the fermentation products; and (d) isolating 20-O-β-D-glucopyranosyl-20(S)-protopanaxadiol from the fermentation products.

When ginsenoside M1 is described as "isolated" or "purified" in the present invention, it should be understood as not absolutely isolated or purified, but relatively isolated or purified. For example, purified ginsenoside M1 refers to one that is more purified compared to its naturally existing form. In one embodiment, a preparation comprising purified ginsenoside M1 may comprise ginsenoside M1 in an amount of more than 50%, more than 60%, more than 70%, more than 80%, more than 90%, or 100% (w/w) of the total preparation. It should be understood that when a certain number was used herein to show a ratio or dosage, said number generally includes that within the range of 10% more and less, or more specifically, the scope of 5% more and less than the number.

The term "individual" or "subject" used herein includes human and non-human animals such as companion animals (such as dogs, cats and the like), farm animals (such as cows, sheep, pigs, horses and the like), or laboratory animals (such as rats, mice, guinea pigs and the like).

The term "treating" as used herein refers to the application or administration of a composition including one or more active agents to a subject afflicted with a disorder, a symptom or conditions of the disorder, a progression of the disorder or at risk of developing the disorder, with the purpose to cure, heal, alleviate, relieve, alter, remedy, ameliorate, improve, or affect the disorder, the symptoms or conditions of the disorder, the disabilities induced by the disorder, or the onset or progression of the disorder.

The term "effective amount" used herein refers to the amount of an active ingredient to confer a desired therapeutic effect in a treated subject. In some embodiments, an effective amount as used herein can be an amount effective in downregulating ACE, upregulating ACE2, degrading angiotensin II, and/or preventing accumulation of angiotensin II. In some embodiments, an effective amount as used herein can be an amount effective in alleviating or reducing damages in organs or tissues caused by accumulation of angiotensin II, for example, pulmonary injuries e.g. alveolitis, lymphocyte infiltration and/or fibrosis in the lung.

The therapeutically effective amount may change depending on various reasons, such as administration route and frequency, body weight and species of the individual receiving said pharmaceutical, and purpose of administration. Persons skilled in the art may determine the dosage in each case based on the disclosure herein, established methods, and their own experience. For example, in certain embodiments, the oral dosage of ginsenoside M1 used in the present invention is 10 to 1,000 mg/kg daily. In some examples, the oral dosage of ginsenoside M1 used in the present invention is 100 to 300 mg/kg daily, 50 to 150 mg/kg daily, 25 to 100 mg/kg daily, 10 to 50 mg/kg daily, or 5 to 30 mg/kg daily. In addition, in some embodiments of the invention, ginsenoside M1 is administered periodically for a certain period of time, for example, daily administration for at least 15 days, one month or two months or longer.

In one embodiment, a therapeutically effective amount of the active ingredient may be formulated with a pharmaceutically acceptable carrier into a pharmaceutical composition of an appropriate form for the purpose of delivery and absorption. Depending on the mode of administration, the pharmaceutical composition of the present invention preferably comprises about 0.1% by weight to about 100% by weight of the active ingredient, wherein the percentage by weight is calculated based on the weight of the whole composition.

As used herein, "pharmaceutically acceptable" means that the carrier is compatible with the active ingredient in the composition, and preferably can stabilize said active ingredient and is safe to the individual receiving the treatment. Said carrier may be a diluent, vehicle, excipient, or matrix to the active ingredient. Some examples of appropriate excipients include lactose, dextrose, sucrose, sorbose, mannose, starch, Arabic gum, calcium phosphate, alginates, tragacanth gum, gelatin, calcium silicate, microcrystalline cellulose, polyvinyl pyrrolidone, cellulose, sterilized water, syrup, and methylcellulose. The composition may additionally comprise lubricants, such as talc, magnesium stearate, and mineral oil; wetting agents; emulsifying and suspending agents; preservatives, such as methyl and propyl hydroxybenzoates; sweeteners; and flavoring agents. The composition of the present invention can provide the effect of rapid, continued, or delayed release of the active ingredient after administration to the patient.

According to the present invention, the form of said composition may be tablets, pills, powder, lozenges, packets, troches, elixers, suspensions, lotions, solutions, syrups, soft and hard gelatin capsules, suppositories, sterilized injection fluid, and packaged powder.

The composition of the present invention may be delivered via any physiologically acceptable route, such as oral, parenteral (such as intramuscular, intravenous, subcutaneous, and intraperitoneal), transdermal, suppository, and intranasal methods. Regarding parenteral administration, it is preferably used in the form of a sterile water solution, which may comprise other substances, such as salts or glucose sufficient to make the solution isotonic to blood. The water solution may be appropriately buffered (preferably with a pH value of 3 to 9) as needed. Preparation of an appropriate parenteral composition under sterile conditions may be accomplished with standard pharmacological techniques well known to persons skilled in the art, and no extra creative labor is required.

According to the present invention, ginsenoside M1 may be used as an active ingredient for treating a disease or condition associated with abnormal balance of ACE and ACE2 or accumulation of angiotensin II in the subject. In some embodiments, such disease or condition includes damages in organs or tissues in the subject such as those in lung, gastrointestinal tract, spleen, lymph nodes, heart, kidney, bladder, liver, gallbladder, adrenal glands and/or testis. In certain examples, the damages are caused by infection of coronavirus, including but not limited to SARS-CoV, MERS-CoV and 2019-nCoV.

In some embodiments, ginsenoside M1 or compositions comprising ginsenoside M1 as the active ingredient may be used in combination with existing therapeutic methods or medicaments, for example, those for treating a disease or condition associated with abnormal balance of ACE and ACE2 or accumulation of angiotensin II as described herein. In one example, ginsenoside M1 is administrated with ACE2. Accordingly, a particular combination of ginsenoside M1 with ACE2 is administrated to a subject in need as described herein.

In some embodiments, ginsenoside M1 or compositions comprising ginsenoside M1 as the active ingredient may be used in combination with corticosteroids (such as prednisolone), non-steriodal anti-inflammatory drugs (NSAIDs), cytotoxic drugs (such as cyclophosphamide, chlorambucil, and azathioprine), immunosuppressants (such as cyclosporine and Mycophenolate Mofetil), and vasodilators (such as angiotensin-converting-enzyme inhibitors (ACE inhibitors)).

In one particular embodiment, ginsenoside M1 is administrated in combination with ACE2 for use treatment of pulmonary injuries, for example, alveolitis, lymphocyte infiltration and/or fibrosis in the lung.

In some embodiments, ginsenoside M1 is administered in combination with an antibiotic, interferon and an anti-viral agent.

In one embodiment, the medicament or therapeutic method used in combination may be used simultaneously (parallel) or sequentially. When medicaments are used in combination, the medicaments may be mixed in the same formula or put in different formulas separately, such as separate capsules, pills, tablets, and injections.

The present invention is further illustrated by the following examples, which are provided for the purpose of demonstration rather than limitation.

### EXAMPLES

Ginsenoside M1, also named Compound K (CK), 20-O-β-D-glucopyranosyl-20(S)-protopanaxadiol (named LCHK168 below), was prepared by methods known in the art, such as those described in Taiwan Patent Application No. 094116005 (I280982) and U.S. Patent No. 7,932,057.

According to previous research, the spike protein (S protein) on coronavirus will bind to ACE2, and the amount of ACE2 will affect the health of the lungs and kidneys (ACE2 Expression in Kidney and Testis May Cause Kidney and Testis Damage After 2019-nCoV Infection https://doi.org/10.1101/2020.02. 12.20022418, Posted February 13, 2020; and Angiotensin-Converting Enzyme 2 Attenuates Bleomycin-Induced Lung Fibrosis in Mice, Cellular Physiology and Biochemistry, 2015; 36: 697-711). This study uses ginsenoside M1 to regulate the content of ACE2 to maintain a healthy body.

Angiotensin II (Ang II)- mediated renal injury represents a major pathogenetic mechanism in most chronic kidney diseases. We found that ginsenoside M1 attenuated Ang II expression by upregulating angiotensin-converting enzyme 2 (ACE2) in the renal tissue. We confirmed this finding in an exogenous Ang II-infused mice model of renal injury, and the results showed consistent in two models. Local renin-angiotensin system (RAS) activation has been shown to play an important role in the pathogenesis of idiopathic pulmonary fibrosis (IPF). It has been reported that ACE2 could inhibit RAS-mediated epithelial injury and fibrogenesis and ACE2 deficiency could aggravate acute and chronic lung injury.

### 1. Material and Methods

### 1.1 Animals and Treatments.

### 1.1.1 Rat (renal damages)

A total of 20 Wistar-Kyoto rats (WKY) and 20 SHR (male, 16-17 weeks old) animals were grouped into four: *WKY* group (8 WKY, orally administered 0.5% CMC-Na); *SHR* group (8 SHR, orally administered 0.5% CMC-Na); *WKY+ Ginsenoside M1* group (8 WKY, orally administered 20mg/kgGinsenoside M1); *SHR + Ginsenoside M1* group (8 SHR, orally administered 20mg/kg Ginsenoside M1). Ginsenoside M1 or placebo administration was carried out once daily for 42 days is was followed by animal sacrifice and blood and renal tissue sample collection. The renal tissue specimens underwent fixation with 4% formalin (histopathology) or were snap-frozen with liquid nitrogen and kept at - 80°Cfor reverse transcription quantitative real-time polymerase chain reaction (RT-qPCR) and enzyme-linked immunosorbent assay (ELISA).

### 1.1.2 Mice (infusion model) (renal damages)

A total of tweenty C57BL/6 mice (male, 10 weeks old) were maintained with rodent chow and water at will. Subcutaneous implantation of a 1002 osmotic minipump was performed at the dorsum of the neck for Ang II (1.5 mg/kg) or normal saline infusion animals were assigned to four groups: *Saline* group (5 mice, infused with normal saline and orally administered 0.5% CMC-Na); *Ang II* group (5 mice, infused with Ang II and orally administered 0.5% CMC-Na); *Saline + Ginsenoside M1* group (5 mice, infused with normal saline and orally administered 20 mg·kg-1·d- 1 Ginsenoside M1); *Ang II + Ginsenoside M1* group (5 mice, infused with Ang II and orally administered 20 mg/kg Ginsenoside M1). Ginsenoside M1 or placebo administration was performed daily for 14 days, followed by animal sacrifice and blood and renal tissue sample collection. The renal tissue specimens underwent fixation with 4% formalin (histopathology) or were snap-frozen with liquid nitrogen and kept at - 80°C (RT-qPCR and ELISA).

### 1.1.3 Mice (pulmonary damages)

The animals were randomly divided into 5 groups as: (1) *Saline* group: 0.9% saline (200 µL) injection via the tail vein; (2) *ACE2* group: a single intraperitoneal injection of ACE2 (0.2 mg/kg); (3) *BLM* group: a single injection of bleomycin at 150 mg/kg via the tail vein; and (4) *BLM+ACE2* group: an injection of bleomycin at 150 mg/kg via the tail vein, followed by an intraperitoneal injection of ACE2 at 0.2 mg/kg. (5) *BLM+ACE2+M1* group: an injection of bleomycin at 150 mg/kg via the tail vein, followed by an intraperitoneal injection of 0.2 mg/kg ACE2 and 60mg/kg M1. After administration, ten animals randomly selected from each group were euthanized on days 7, 14 and 28 by cervical dislocation.

### 1.2 Histopathological Assessment.

Renal tissue specimens underwent fixation with 4% formalin, paraffin embedding, sectioning at 4 µm, and staining with hematoxylin and eosin (H&E) and Masson trichrome stain, respectively. Then, a Nikon E100 light microscope was employed for analysis.

### 1.3 Immunohistochemistry (IHC)

ACE and ACE2 primary antibodies were produced by Bioss Antibodies. Peroxidase-linked goat anti-rabbit secondary antibodies and the DAB and two-step rabbit IHC kits were provided by ZSGB-BIO. IHC was carried out as proposed by ZSGB-BIO.

### 1.4 Ang II and Ang-(1-7) Level Assessment in the Renal Tissue

The supernatants of renal specimens were prepared as follows. A total of 100 mg of the renal tissue was homogenized in 900 µL of ice-cold normal saline and submitted to centrifugation (1000 g, 4°C for 15 min). The resulting supernatants were kept at - 80°C until analysis with Ang II and Ang-(1-7) ELISA kits, respectively, in accordance with the manufacturer's protocols.

### 2. Results

### 2.1 Ginsenoside M1 Reduces Ang II Levels in the Renal Tissue in SHR

There are two main ACE which regulate the levels of Ang II *in vivo,* including ACE and ACE2. ACE transforms angiotensin I into Ang II, and ACE2 transforms Ang II into Ang-(1-7). So upregulation of ACE induces Ang II increase, while ACE2 upregulation attenuates Ang II increase. Spontaneously hypertensive rat (SHR) is an animal model of genetic hypertension. According to ELISA data (Figure 1), Ang II levels in the renal tissue of the *SHR* group were significantly higher than those of *WKY* and *WKY+ Ginsenoside M1* groups. Treatment with Ginsenoside M1 significantly reduced Ang II levels in the renal tissue in SHR, while showing limited effects in the *WKY* group. Ang-(1-7) amounts in the *SHR* group were significantly higher than those of the *WKY* and *WKY+ Ginsenoside M1* groups, with the SHR + Ginsenoside M1 showing even significantly higher values compared with the *SHR* group. IHC results (Figure 2) showed that ACE levels were markedly elevated in the two *SHR* groups compared with the two *WKY* groups. ACE2 amounts in the renal tissue were compensatorily upregulated in the *SHR* group, compared with the *WKY* and *WKY+ Ginsenoside M1* groups. Meanwhile, Ginsenoside M1 treatment could further upregulate ACE2 in the renal tissue in SHR.is was the main mechanism that Ginsenoside M1 treatment downregulated Ang II and upregulated Ang-(1-7) in the kidneys of SHR. Meanwhile, Ginsenoside M1 had no significant effect on ACE expression in either WKY or SHR.

### 2.2 Ginsenoside M1 Reduces Ang II Levels in the Renal Tissue in Mice Infused Ang II

According to ELISA data (Figure 3), Ang II infusion significantly raised Ang II levels in the renal tissue in mice, and Ginsenoside M1 treatment could attenuate this increase significantly, although Ang II levels in the *Ang II + Ginsenoside M1* group were still significantly higher than those of the two groups infused saline. Meanwhile, Ang-(1-7) levels in the *Ang II* group were significantly higher than those of the *Saline* and *Saline + Ginsenoside M1* groups; the *Ang II + Ginsenoside M1* group showed a significant increase compared with the *Ang II* group. IHC data (Figure 4) showed no marked differences in various group pairs in ACE expression. ACE2 expression levels in the *Ang II + Ginsenoside M1* group were markedly elevated compared with those of the other three groups, which might be the main mechanism for degrading exogenous angiotensin.

### 2.3 Treatment of alveolitis and fibrosis by M1 and ACE2

As shown in Figure 5, a single injection of bleomycin at the dose of 150 mg/kg via the tail vein in mice induced mild to moderate alveolitis on day 7. Administration of bleomycin is a well-characterized model of pulmonary alveolitis or fibrosis in mice. The fibrotic changes in the lungs were determined using the Szapiel scoring method, higher score indicating greater disease condition. Lungs exhibited focal distribution of alveolitis that was predominantly subpleural and perivascular, in which the alveolar septa were mildly thickened with edema and inflammatory cell infiltration; no apparent fibrosis was observed. At the same time, administration of ACE2 did not significantly alter the pathological changes on day 7. On day 14, the BLM group showed disease progression. Lungs exhibited moderate to severe alveolitis and moderate fibrosis with patchy inflammatory consolidation and collagen deposition, while the administration of ACE2 significantly attenuated both alveolitis and fibrosis. On day 28, in the BLM group, alveolitis spontaneously resolved; however, derangement of the alveolar architecture continued, and fibrosis persisted, characterized by diffuse, dense, thick collagen bundles and fibroblast foci. ACE2 noticeably reflected a long-term therapeutic effect on day 28, with sections in the BLM+ACE2 group presenting only mild to moderate alveolitis, with sections in the BLM+ACE2+M1 group presenting only mild alveolitis and significant attenuation of fibrosis . No signs of alveolitis or fibrosis were found in the Saline group or the ACE2 group among the different time points.

### References

1. Journal of Molecular Medicine/October 2006, Volume 84, Issue 10, pp 814-820| Cite as/Lessons from SARS: control of acute lung failure by the SARS receptor ACE2. Pharmacology & Therapeutics/Volume 128, Issue 1, October 2010, Pages 119-128/Trilogy of ACE2: A peptidase in the renin-angiotensin system, a SARS receptor, and a partner for amino acid transporters.
2. Published in final edited form as:Annu Rev Virol. 2016 September 29; 3(1): 237-261. doi:10.1146/annurev-virology-110615-042301./ Structure, Function, and Evolution of Coronavirus Spike Proteins/ Fang Li Department of Pharmacology, University of Minnesota Medical School, Minneapolis, Minnesota 55455
3. Antioxidant Treatment and Alcoholism/Camila S. Silva PhD, ... Helio Vannucchi MD, PhD, in Molecular Aspects of Alcohol and Nutrition, 2016
4. Beta-Glucosidase From Penicillium/Gustavo Molina, ... Gláucia M. Pastore, in New and Future Developments in Microbial Biotechnology and Bioengineering, 2018
5. Ginseng and Gastrointestinal Protection*/Min-Hyun Kim, Hyeyoung Kim, in Gastrointestinal Tissue, 2017

## Claims

1. Ginsenoside M1 for use in the treatment of damages in organs or tissues in a subject matter caused by infection with coronavirus, wherein the ginsenoside M1 is used as a modulator of an angiotensin regulating enzyme in a subject in need thereof.

2. The ginsenoside M1 for use according to claim 1, wherein the angiotensin regulating enzyme is selected from the group consisting of angiotensin-converting enzyme (ACE) and angiotensin-converting enzyme 2 (ACE2).

3. The ginsenoside M1 for use according to claim 1 or 2, wherein the modulator is an ACE inhibitor or an ACE2 activator or both.

4. The ginsenoside M1 for use according to claim 3, wherein the modulator is effective in degrading angiotensin II and preventing accumulation of angiotensin II in the subject.

5. The ginsenoside M1 for use according to claim 1, wherein the coronavirus is selected from severe acute respiratory syndrome coronavirus (SARS-CoV), middle east respiratory syndrome coronavirus (MERS-CoV), and novel coronavirus (2019-nCoV).

6. The ginsenoside M1 for use according to claim 1, wherein the ginsenoside M1 is administered in combination with ACE2.

7. The ginsenoside M1 for use according to claim 1, wherein the ginsenoside M1 is administered in combination with one or more additional therapeutic agents selected from the group consisting of corticosteroids, non-steroidal anti-inflammatory drugs (NSAIDs), cytotoxic drugs, immunosuppressants, and vasodilators.

8. The ginsenoside M1 for use according to claim 7, wherein the corticosteroid is prednisolone; the cytotoxic drug is cyclophosphamide, chlorambucil, or azathioprine; the immunosuppressant is cyclosporine or Mycophenolate Mofetil; and the vasodilator is angiotensin-converting-enzyme inhibitors.

9. The ginsenoside M1 for use according to claim 1, wherein the ginsenoside M1 is administered in combination with one or more additional therapeutic agents selected from the group consisting of an antibiotic, interferon and an anti-viral agent.

## Patentansprüche

1. Ginsenosid M1 zur Verwendung bei der Behandlung von Schäden in Organen oder Geweben in einem Patienten, die durch eine Infektion mit dem Coronavirus verursacht werden, wobei das Ginsenosid M1 als Modulator eines angiotensinregulierenden Enzyms in einem Patienten, der dies benötigt, verwendet wird.

2. Ginsenosid M1 zur Verwendung nach Anspruch 1, wobei das angiotensinregulierende Enzym aus der Gruppe ausgewählt ist, die aus Angiotensin-konvertierendem Enzym (ACE) und Angiotensin-konvertierendem Enzym 2 (ACE2) besteht.

3. Ginsenosid M1 zur Verwendung nach Anspruch 1 oder 2, wobei der Modulator ein ACE-Inhibitor oder ein ACE2-Aktivator oder beides ist.

4. Ginsenosid M1 zur Verwendung nach Anspruch 3, wobei der Modulator wirksam ist beim Abbau von Angiotensin II und beim Verhindern einer Akkumulation von Angiotensin II in dem Patienten.

5. Ginsenosid M1 zur Verwendung nach Anspruch 1, wobei das Coronavirus ausgewählt ist aus dem Coronavirus des schweren akuten respiratorischen Syndroms (SARS-CoV), dem Nahost-Atemwegssyndrom-Coronavirus (MERS-CoV) und dem neuen Coronavirus (2019-nCoV).

6. Ginsenosid M1 zur Verwendung nach Anspruch 1, wobei das Ginsenosid M1 in Kombination mit ACE2 verabreicht wird.

7. Ginsenosid M1 zur Verwendung nach Anspruch 1, wobei das Ginsenosid M1 in Kombination mit einem oder mehreren zusätzlichen therapeutischen Mitteln verabreicht wird, ausgewählt aus der Gruppe bestehend aus Kortikosteroiden, nicht-steroidalen entzündungshemmenden Medikamenten (NSAIDs), zytotoxischen Medikamenten, Immunsuppressiva und Vasodilatatoren.

8. Ginsenosid M1 zur Verwendung nach Anspruch 7, wobei das Kortikosteroid Prednisolon ist; das zytotoxische Medikament Cyclophosphamid, Chlorambucil oder Azathioprin ist; das Immunsuppressivum Cyclosporin oder Mycophenolat Mofetil ist; und der Vasodilatator ein Angiotensin-konvertierender Enzyminhibitor ist.

9. Ginsenosid M1 zur Verwendung nach Anspruch 1, wobei das Ginsenosid M1 in Kombination mit einem oder mehreren zusätzlichen therapeutischen Mitteln verabreicht wird, ausgewählt aus der Gruppe bestehend aus einem Antibiotikum, Interferon und einem antiviralen Mittel.

## Revendications

1. Ginsénoside M1 utilisé pour le traitement des dommages causés aux organes ou aux tissus d'un sujet par une infection par un coronavirus, dans lequel le ginsénoside M1 est utilisé comme modulateur d'une enzyme régulatrice de l'angiotensine chez un sujet qui en a besoin.

2. Ginsénoside M1 utilisé selon la revendication 1, dans lequel l'enzyme régulatrice de l'angiotensine est choisie dans le groupe constitué de l'enzyme de conversion de l'angiotensine (ECA) et de l'enzyme de conversion de l'angiotensine 2 (ECA2).

3. Ginsénoside M1 utilisé selon la revendication 1 ou 2, dans lequel le modulateur est un inhibiteur de l'ECA ou un activateur de l'ECA2 ou les deux.

4. Ginsénoside M1 utilisé selon la revendication 3, dans lequel le modulateur est efficace pour dégrader l'angiotensine Il et prévenir l'accumulation d'angiotensine Il chez le sujet.

5. Ginsénoside M1 utilisé selon la revendication 1, dans lequel le coronavirus est choisi parmi le coronavirus du syndrome respiratoire aigu sévère (SARS-CoV), le coronavirus du syndrome respiratoire du Moyen-Orient (MERS-CoV) et le nouveau coronavirus (2019-nCoV).

6. Ginsénoside M1 utilisé selon la revendication 1, dans lequel le ginsénoside M1 est administré en association avec l'ECA2.

7. Ginsénoside M1 utilisé selon la revendication 1, dans lequel le ginsénoside M1 est administré en association avec un ou plusieurs agents thérapeutiques supplémentaires choisis dans le groupe constitué par les corticostéroïdes, les anti-inflammatoires non stéroïdiens (AINS), les médicaments cytotoxiques, les immunosuppresseurs et les vasodilatateurs.

8. Ginsénoside M1 utilisé selon la revendication 7, dans lequel le corticostéroïde est la prednisolone ; le médicament cytotoxique est le cyclophosphamide, le chlorambucil ou l'azathioprine ; l'immunosuppresseur est la cyclosporine ou le mycophénolate mofétil ; et le vasodilatateur est un inhibiteur de l'enzyme de conversion de l'angiotensine.

9. Ginsénoside M1 utilisé selon la revendication 1, dans lequel le ginsénoside M1 est administré en association avec un ou plusieurs agents thérapeutiques supplémentaires choisis dans le groupe constitué d'un antibiotique, d'un interféron et d'un agent antiviral.
